# EUROPEAN PATENT APPLICATION

(11) **EP 4 623 983 A1**
(43) Date of publication of application: **01.10.2025**
(21) Application number: 23894452.4
(22) Date of filing: 10.11.2023
(51) Int. Cl.: A61M 39/26

(54) **NEEDLELESS CONNECTOR**

(30) Priority: 24.11.2022 JP 2022187592
(71) Applicant: Nipro Corporation, Osaka 566-8510 (JP)
(72) Inventor: YAMAGUCHI, Takeshi, Settsu-shi, Osaka 566-8510 (JP); YAMAGATA, Minami, Settsu-shi, Osaka 566-8510 (JP); SHIMAMOTO, Jumpei, Settsu-shi, Osaka 566-8510 (JP)
(74) Representative: Germain Maureau
(86) International application number: PCT/JP2023/040632
(87) International publication number: WO 2024/111443

(57) **Abstract**

A needleless connector includes a housing 103 and a plunger valve 101 housed in the housing. The plunger valve 101 has a cylindrical wall section 113, a head section 111 formed closer to an insertion port than is the cylindrical wall section 113, and a middle section 112 formed between the head section 111 and the cylindrical wall section 113. The plunger valve 101 is configured to be deformed such that the volume of a flow path increases, when a male connector is inserted, and restore such that the volume of the flow path decreases, when a male connector is removed. The head section 111 has a notched groove 151 having a radial depth that gradually becomes smaller from a top vertex portion thereof toward the middle section 112.

## Description

### TECHNICAL FIELD

The present disclosure relates to needleless connectors.

### BACKGROUND ART

To patients receiving an infusion or blood transfusion, additional medicinal liquids or the like may be administered. To this end, a fluid circuit is provided with a port, and a medicinal liquid or the like is put into the fluid circuit through the port. Although there are various kinds of ports that are provided to a fluid circuit, it is needleless connectors that are widely used. The needleless connector is opened to be in communication with the circuit when a male luer connector is inserted therein, and is closed when the male luer connector is removed therefrom. Ports employing a needleless connector can avoid the risk of needle-stick accidents, which are likely to occur when puncture ports are used, and the risk of erroneous switching operations, which are likely to occur when three-way stopcocks or the like are used.

However, for ports employing a needleless connector, when the male luer connector is removed, the internal pressure may decrease, so that a liquid such as blood or medicinal liquid in the main flow path is likely to be drawn into the needleless connector.

In order to cause the liquid to be less likely to be drawn into the needleless connector, a needleless connector has been studied which includes a collapsible valve that is deformed when a male luer connector is inserted and restores when the male luer connector is removed (see, for example, Patent Document 1). When a male luer connector is inserted into the needleless connector, so that the collapsible valve is deformed, a flow path is formed in the needleless connector. When the male luer connector is removed, so that the valve restores, the flow path becomes smaller, and therefore, the medicinal liquid in the flow path is pushed toward the tip end. As a result, it can be expected that the medicinal liquid drawn into the needleless connector is reduced.

### CITATION LIST

### PATENT DOCUMENT

PATENT DOCUMENT 1: Japanese Unexamined Patent Publication (Japanese Translation of PCT Application) No. 2013-500128

### SUMMARY OF THE INVENTION

### TECHNICAL PROBLEM

However, the needleless connector including a collapsible valve has a narrower internal flow path, which has a greater flow path drag, and therefore, has problems that high pressure is needed for injection of a medicinal liquid, and that the flow rate decreases. When a medicinal liquid is injected by free fall like drip infusion, high pressure cannot be applied, which particularly causes a problem. Even when a medicinal liquid is injected using a syringe, the increased drag is still problematic.

In addition, the collapsible valve is likely to produce a space in the needleless connector in which the medicinal liquid easily stagnates, and the medicinal liquid that has entered such a space is likely to continue to remain in the needleless connector.

Furthermore, the collapsible valve is required to smoothly deform and move when the male connector is inserted, and smoothly restore when the male connector is removed. Therefore, it is desirable to provide a guide mechanism for the collapsible valve in the insertion port. The collapsible valve may be configured to have not only the plunger valve's function of pushing the liquid or the like toward the tip end, but also the function of closing the insertion port, into which the male connector is inserted, in a manner that allows the insertion port to be openable and closeable. A slit valve that closes the insertion port may be provided separately from the plunger valve. In that case, the insertion port needs to be provided with the guide mechanism for the collapsible valve and the detachment prevention mechanism for the slit valve, disadvantageously leading to an increase in the diameter and height of the insertion port.

The present disclosure discloses implementations that reduce the liquid drawn into the needleless connector and overcome at least one of the above problems.

### SOLUTION TO THE PROBLEM

A first embodiment of a needleless connector according to the present disclosure includes: a cylindrical housing having, at an end portion thereof, an insertion port into which a male connector is inserted; and a plunger valve housed in the housing. A flow path is formed between an inner wall surface of the housing and an outer wall surface of the plunger valve. The plunger valve has a cylindrical wall section, a head section formed closer to the insertion port than is the cylindrical wall section, and a middle section formed between the head section and the cylindrical wall section. The plunger valve is configured to be deformed such that the volume of the flow path increases, when a male connector is inserted in the insertion port, and restore such that the volume of the flow path decreases, when a male connector inserted in the insertion port is removed. The flow path has an upstream flow path formed at the position of the head section, a downstream flow path formed at the position of the cylindrical wall section, and a middle flow path connecting the upstream flow path and the downstream flow path together. The head section has a notched groove having a radial depth that gradually becomes smaller from a top vertex portion thereof toward the middle section.

In the first embodiment of the needleless connector, when a male connector inserted in the insertion port is removed, a medicinal liquid or the like in the needleless connector is pushed out toward the tip end because the plunger valve restores such that the volume of the flow path decreases. As a result, the liquid drawn into the needless connector can be reduced. In addition, the plunger valve has, at the head section, the notched groove having a radial depth that gradually becomes smaller from the top vertex portion toward the middle section. Therefore, the volume of the flow path on the top vertex portion side can be ensured, and the flow path drag can be reduced. A medicinal liquid or the like injected from a male connector inserted in the insertion port can be quickly guided into the notched groove, resulting in a smooth flow of the medicinal liquid, and therefore, the stagnation is less likely to occur.

In the first embodiment of the needleless connector, the notched groove can be formed to have a circumferential width that gradually become smaller from the top vertex portion toward the middle section. This structure can reduce the flow path drag in the insertion port, and reduce the dead volume.

A second embodiment of a needleless connector according to the present disclosure includes: a cylindrical housing having, at an end portion thereof, an insertion port into which a male connector is inserted; and a plunger valve housed in the housing. A flow path is formed between an inner wall surface of the housing and an outer wall surface of the plunger valve. The plunger valve has a cylindrical wall section, a head section formed closer to the insertion port than is the cylindrical wall section, and a middle section formed between the head section and the cylindrical wall section. The plunger valve is configured to be deformed such that the volume of the flow path increases, when a male connector is inserted in the insertion port, and restore such that the volume of the flow path decreases, when a male connector inserted in the insertion port is removed. The flow path has a portion in which a liquid flows circumferentially.

In the second embodiment of the needleless connector, the liquid drawn into the needleless connector can be reduced, and the needleless connector has a portion that actively guides the liquid in the circumferential direction. Regions in which the liquid flows can be likely to be equalized, and therefore, the occurrence of pockets of the stagnating liquid can be reduced.

In the second embodiment of the needleless connector, the flow path may have an upstream flow path formed at the position of the head section, a downstream flow path formed at the position of the cylindrical wall section, and a middle flow path connecting the upstream flow path and the downstream flow path together. The upstream flow path and the downstream flow path may be separated from each other circumferentially. This structure allows the liquid to flow circumferentially in the middle flow path, and therefore, the stagnation is even less likely to occur.

In the second embodiment of the needleless connector, the head section may have a notched groove serving as the upstream flow path. The housing may have a housing groove serving as the downstream flow path at a portion thereof opposite the cylindrical wall section. The plunger valve may be housed in the housing with the notched groove and the housing groove separated from each other circumferentially. This structure easily provides a portion in which the liquid flows circumferentially.

A third embodiment of a needleless connector according to the present disclosure includes: a cylindrical housing having, at an end portion thereof, an insertion port into which a male connector is inserted; a plunger valve housed in the housing; and a slit valve arranged closer to the insertion port than is the plunger valve and configured to block an opening of the insertion port. A flow path is formed between an inner wall surface of the housing and an outer wall surface of the plunger valve. The plunger valve has a cylindrical wall section, a head section formed closer to the insertion port than is the cylindrical wall section, and a middle section formed between the head section and the cylindrical wall section. The plunger valve is configured to be deformed such that the volume of the flow path increases, when a male connector is inserted in the insertion port, and restore such that the volume of the flow path decreases, when a male connector inserted in the insertion port is removed. The slit valve has a middle section having a slit and configured to block the opening of the insertion port in a manner that allows the insertion port to be openable and closeable, and a skirt-shaped section formed on an outer peripheral side of the middle section and protruding from the middle section toward the plunger valve. The housing has, at the insertion port, an outer cylindrical part and an inner cylindrical part. The inner cylindrical part, together with the outer cylindrical part, sandwiches the skirt-shaped section, and is positioned between the skirt-shaped section and the head section and configured to guide movement of the head section.

In the third embodiment of the needleless connector, the inner cylindrical part of the insertion port serves both as a detachment prevention mechanism for the slit valve and a guide mechanism for the plunger valve. Because the slit valve having the skirt-shaped section is sandwiched between the outer cylindrical part and inner cylindrical part of the insertion port, the slit valve can be less likely to be detached without the need of an increase in the outer shape of the slit valve compared to when the slit valve is simply sandwiched vertically. In addition, the inside of the inner cylindrical part serves as a region in which the plunger valve is moved, and therefore, an increase in the size in the height direction can be reduced, and the deformation, movement, and restoration of the plunger valve can be guided and thereby smoothly performed.

### ADVANTAGES OF THE INVENTION

The needleless connector according to the present disclosure can overcome at least one of various problems with conventional needleless connectors.

### BRIEF DESCRIPTION OF THE DRAWINGS

[FIG. 1] FIG. 1 is a perspective view illustrating a needleless connector according to an embodiment.
[FIG. 2] FIG. 2 is a cross-sectional view taken along line II-II of FIG. 1.
[FIG. 3] FIG. 3 is a cross-sectional view taken along line III-III of FIG. 1.
[FIG. 4] FIG. 4 is a view illustrating the cross-section of FIG. 2 in a state in which a male connector is inserted.
[FIG. 5] FIG. 4 is a view illustrating the cross-section of FIG. 3 in a state in which a male connector is inserted.
[FIG. 6] FIG. 6 is a side view illustrating a plunger valve.
[FIG. 7] FIG. 7 is a cross-sectional view illustrating a plunger valve.
[FIG. 8] FIG. 8 is a top view illustrating a plunger valve.

### DESCRIPTION OF EMBODIMENTS

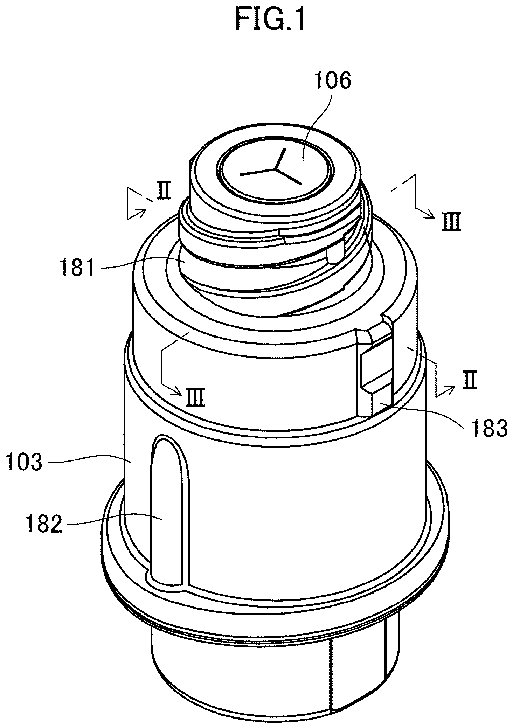

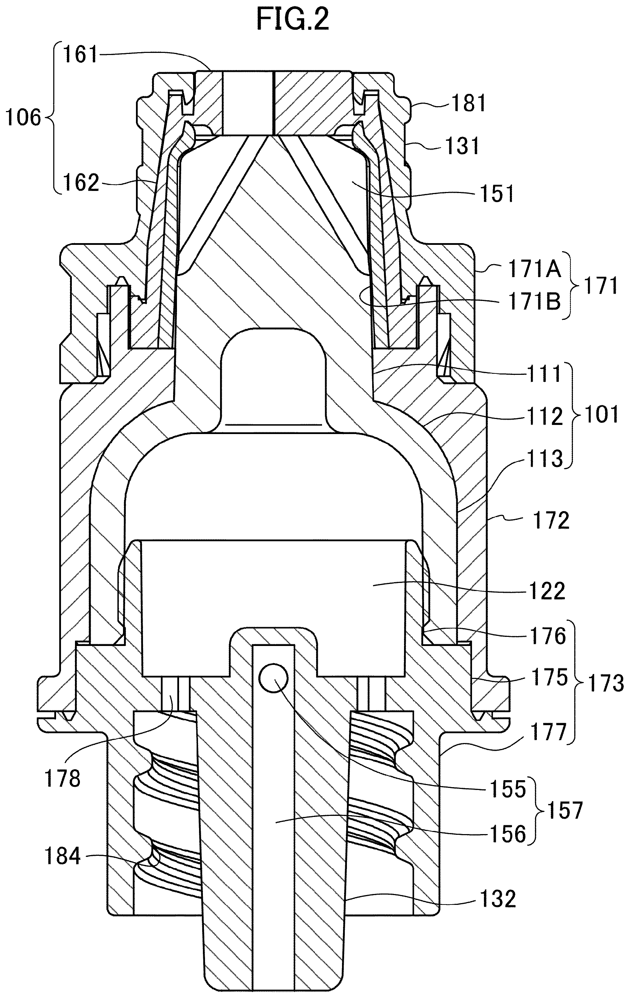

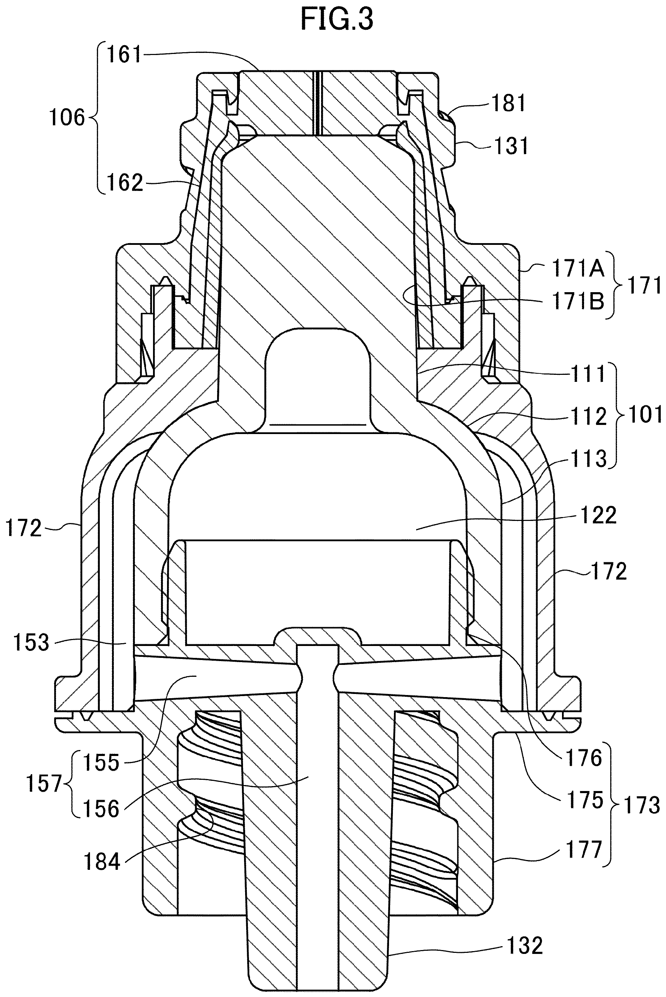

As illustrated in FIGS. 1-3, a needleless connector according to an embodiment has a cylindrical housing 103, and a plunger valve 101 that is housed in the housing 103. An insertion port 131 into which a male connector is inserted is formed at an upper portion of the housing, and an outlet port 132 that is a male connector is formed at a lower portion of the housing. The insertion port 131 is closed by a slit valve 106 in a manner that allows the insertion port 131 to be openable and closeable. Although the direction in which the needleless connector is oriented in use is not particularly limited, in the following description the side on which the insertion port 131 is positioned is the upper side.

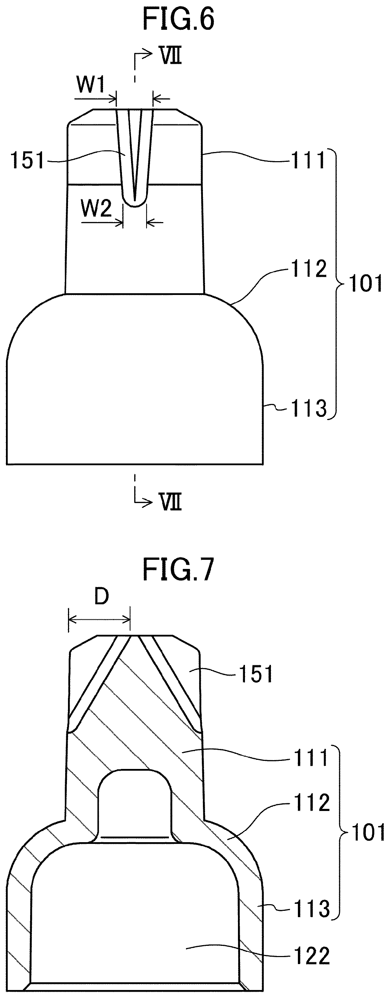

As illustrated in FIGS. 6-8, the plunger valve 101 has a head section 111, a cylindrical wall section 113, and a middle section 112 formed between the head section 111 and the cylindrical wall section 113. The outer diameter of the head section 111 is smaller than that of the cylindrical wall section 113. The middle section 112, which connects the head section 111 and the cylindrical wall section 113 together, has a gently curved surface that is convex upward.

The plunger valve 101 is roughly in the shape of a bell having an open lower end. A cavity 122 is formed in the plunger valve 101, extending from the lower end of the cylindrical wall section 113 to a lower portion of the head section 111. The head section 111 has a solid upper portion. A notched groove(s) 151 is formed in the outer surface of the head section 111. The notched groove 151 is an inclined groove whose radial depth D gradually becomes smaller from the top vertex of an upper end portion of the head section 111 toward the middle section 112.

The plunger valve 101 is housed in an accommodation space formed in the housing 103. The accommodation space has substantially the same shape as the outer shape of the plunger valve 101 when the plunger valve 101 is not deformed. An upper portion of the accommodation space serves as the insertion port 131, into which a male connector is inserted. A housing groove(s) 153 that extends vertically is formed in a portion of the inner wall of the housing 103 that is opposite the cylindrical wall section 113. A supporting wall 176 that is in upright position is circumferentially formed at a lower end portion of the accommodation space of the housing 103. A lower portion of the cylindrical wall section 113 is sandwiched between the inner wall of the accommodation space and the supporting wall 176.

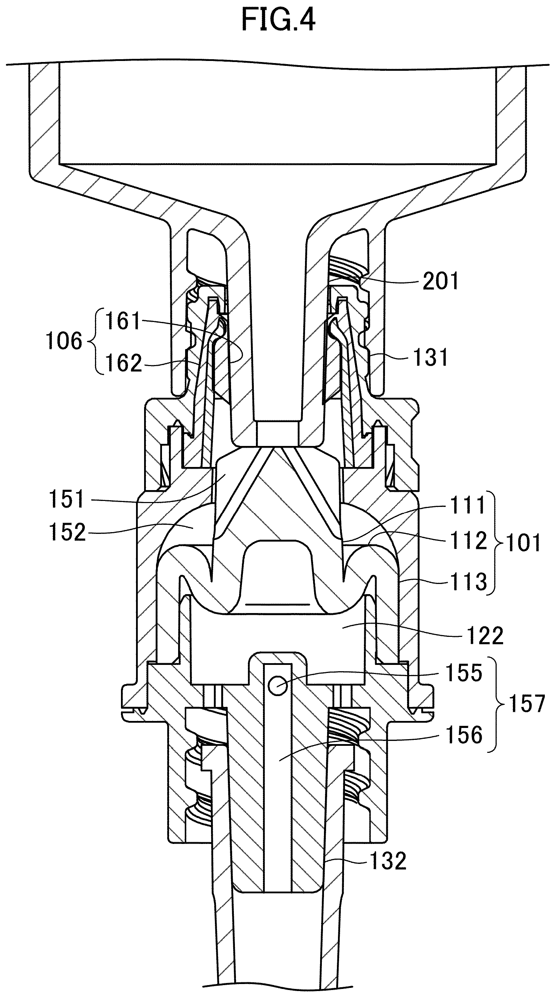

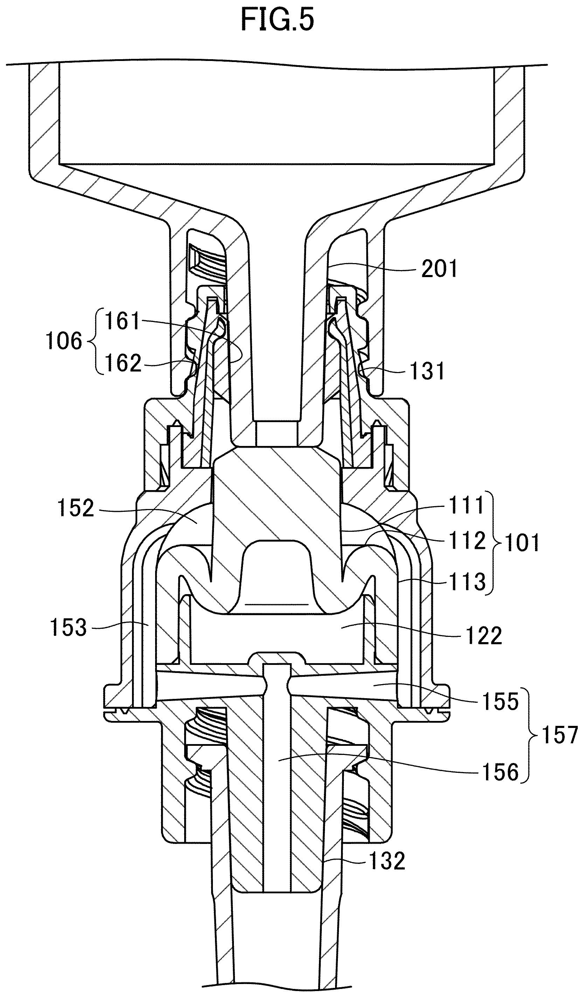

As illustrated in FIGS. 4 and 5, when a male connector 201 is inserted from the insertion port 131, the male connector 201 pushes the head section 111 of the plunger valve 101 downward. The plunger valve 101, which is formed of an elastic material such as rubber or elastomer, is deformed when pressed. The hollow middle section 112 is greatly deformed to fold into the inner cavity 122, while the solid upper portion of the head section 111 and the cylindrical wall section 113, which is held by the supporting wall 176, are not substantially deformed.

When the middle section 112 is deformed, the head section 111 is moved downward, and a middle section space 152 that circumferentially extends is formed between the outer surface of the middle section 112 and the inner surface of the accommodation space. The middle section space 152 connects the notched groove 151 formed in the head section 111 and the housing groove 153 together. As a result, a plunger section flow path including the notched groove 151 as an upstream flow path, the middle section space 152 as a middle flow path, and the housing groove 153 as a downstream flow path is opened between the plunger valve 101 and the housing 103.

A lower end portion of the housing groove 153 is connected to a housing lower portion flow path 157 including a radial flow path 155 that extends toward the center of the housing 103 and an axial flow path 156 that penetrates through the outlet port 132, which is a male connector. Therefore, the plunger section flow path and the housing lower portion flow path 157 form a flow path connecting the insertion port 131 and the outlet port 132 together.

When the male connector 201 is pulled out of the insertion port 131, the plunger valve 101 restores. As a result, the middle section space 152 becomes smaller. Therefore, the volume of the plunger section flow path is reduced, and therefore, a liquid in the flow path flows out from the outlet port 132, so that the liquid is less likely to be drawn into the insertion port 131 when the male connector 201 is pulled out of the insertion port 131. It should be noted that when the male connector 201 is not inserted in the insertion port 131, the middle section 112 of the plunger valve 101 and the inner wall of the accommodation space do not need to contact without any clearance to produce a liquid-tight state, or alternatively, may be configured to produce a liquid-tight state.

As illustrated in FIGS. 6-8, the notched groove 151, which is the upstream flow path, is an inclined groove whose radial depth D gradually becomes smaller from the top vertex portion toward the middle section 112. Therefore, the volume of a portion of the upstream flow path in the vicinity of the top vertex portion, which is brought into contact with the male connector 201, can be particularly greater than when the upstream flow path is formed by a groove having a uniform depth along the surface of the head section 111, resulting in a reduction in the flow path drag. The inclined groove having a generally triangular cross-sectional shape has a total volume smaller than that of a groove having a generally quadrangular cross-sectional shape and therefore having a uniform radial depth D. However, the flow of the liquid moving downward from the male connector 201 can be directed in the radial direction without being bent, whereby an increase in pressure loss can be reduced. The stagnation can also be reduced. Although the radial depth D of the notched groove 151 becomes smaller toward the middle section 112 (downward), the flow path drag does not significantly increase, because a lower end portion of the notched groove 151 is integrated with the middle section space 152, which is formed inside the middle section 112. Furthermore, compared to the case in which a groove having a generally quadrangular cross-sectional shape is formed, the strength of the head section 111 can be maintained and therefore the head section 111 is less likely to collapse.

As illustrated in FIG. 6, the notched groove 151 is preferably formed such that a circumferential width W1 at the top vertex portion is greater than a circumferential width W2 at the middle section 112, and the circumferential width thereof becomes smaller smoothly (without a step). As a result, the liquid flowing in the notched groove 151 can be accelerated to smoothly flow into the middle section space 152, which is the middle section flow path. In addition, the side and bottom surfaces of the notched groove 151 are preferably connected to each other without an acute-angled portion being produced. If the side and bottom surfaces are smoothly connected together, the stagnation can be further reduced.

In this embodiment, in a top view of the head section 111, two notched grooves 151 are formed, extending from a center portion toward an outer peripheral portion of the head section 111, and are separated from each other by 180° circumferentially. Because the two notched grooves 151 are provided, the flow rate can be ensured, and a decrease in the strength of the head section 111 can be reduced, which allows the head section 111 to be less likely to deform when the male connector 201 is inserted. In addition, because the two notched grooves 151 are separated from each other by 180°, the flows of the liquid are likely to be equalized. It should be noted that the number of the notched grooves 151 may be one or three or more. In the case in which a single notched groove 151 is provided, the notched groove 151 may be configured to extend from an outer peripheral portion of the head section 111 through a middle portion thereof to an outer peripheral portion on the opposite side thereof. It should be note that even in the case in which a single notched groove 151 is provided, then if the groove is formed so as to have a great flow path area while a decrease in the strength of the head section 111 is reduced, an increase in the flow path drag can be avoided.

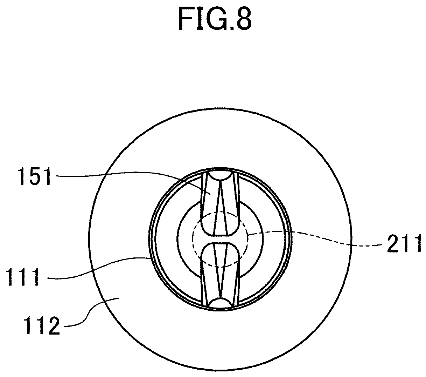

In the case in which a plurality of notched grooves 151 are formed, extending from a center portion to an outer peripheral portion of the head section 111, it is preferable that as illustrated in FIG. 8, the notched groove 151 should greatly enter the inside of an inner radial range 211 of the male connector 201. This allows the opening of the tip end of the male connector 201 not to be blocked, and therefore, the liquid can flow out without being dragged and after flowing out, can directly flow into the notched groove 151, resulting in a smoother flow of the liquid.

In this embodiment, the plunger valve 101 is housed in the housing 103 such that the notched groove 151 and the housing groove 153 are separated from each other circumferentially. Because the notched groove 151 and the housing groove 153 are separated from each other circumferentially, the middle section space 152 functions as a flow path in which the liquid flows circumferentially, but not as a pool in which the liquid spreads circumferentially and stagnates. Therefore, the liquid can smoothly flow in the flow path, and therefore, the stagnation is less likely to occur.

The magnitude of the circumferential separation between the notched groove 151 and the housing groove 153 can be determined based on the number of notched grooves 151 and the number of housing grooves 153. In the case in which two notched grooves 151 are provided and separated from each other by 180° and two housing grooves 153 are provided and separated from each other by 180°, the magnitude of the circumferential separation between the notched groove 151 and the housing groove 153 is preferably 45-135° in order to reduce the stagnation by increasing the separation to some extent. The present disclosure is not limited to this.

In this embodiment, the housing 103 has a protrusion section 182 protruding radially outward, and the housing groove 153 is formed at the position of the protrusion section 182. Because the housing groove 153 is formed at the position of the protrusion section 182, the wall thickness of the housing 103 at the position of the housing groove 153 can be substantially the same as that of the other portion, and therefore, a decrease in the strength of the housing 103 can be reduced. In addition, the occurrence of a distortion during molding can be reduced.

In this embodiment, a positioning projection 183 is formed at a position separated by 90° circumferentially from the protrusion section 182, at which the housing groove 153 is formed in the housing 103. When the plunger valve 101 is attached to the housing 103, then if the position of the notched groove 151 of the plunger valve 101 is aligned with the position of the positioning projection 183, the attachment can be facilitated. Instead of the positioning projection 183 being provided separately, the protrusion section 182 may be used as a positioning marker.

In this embodiment, the housing 103 is formed by combining an insertion port housing 171, a middle housing 172, and an outlet port housing 173. The outlet port housing 173 has a disc-shaped base section 175, the outlet port 132, which is a male connector protruding from the base section 175, and a coupler 177 surrounding the outlet port 132. The middle housing 172 has a cylindrical shape that is fitted on the outer surface of the base section 175 of the outlet port housing 173. The insertion port housing 171 has a cylindrical shape that is fitted on the outer surface of an upper end portion of the middle housing 172.

The base section 175 of the outlet port housing 173 closes the opening of the lower end of the accommodation space formed by the middle housing 172 and the insertion port housing 171. The base section 175 has the supporting wall 176, which has a cylindrical shape protruding upward. A lower end portion of the plunger valve 101 is fitted on the outer surface of the supporting wall 176, and is sandwiched between the outer surface of the supporting wall 176 and the inner surface of the middle housing 172. As a result, the liquid flowing in a flow path formed between the outer surface of the plunger valve 101 and the inner surface of the housing 103 does not enter the cavity 122 of the plunger valve 101, which is a liquid-tight state.

The base section 175 has a ventilation hole 178 through which the cavity 122 of the plunger valve 101 is in communication with the outside of the housing 103. When the plunger valve 101 is pressed and deformed, air in the cavity 122 is discharged through the ventilation hole 178, and when the plunger valve 101 restores, air flows through the ventilation hole 178 into the cavity 122. Therefore, the deformation and restoration of the plunger valve 101 are smoothly performed. The shape and size of the ventilation hole 178 are not particularly limited. The ventilation hole 178 may have various structures. In addition, although in this embodiment, the ventilation hole 178 has an opening between the outlet port 132 and the coupler 177, the opening may be positioned outward of the coupler 177.

The insertion port housing 171 has an outer cylindrical part 171A and an inner cylindrical part 171B. A male threaded portion 181 that is screwed to the coupler of the male connector is formed on the outer surface of the outer cylindrical part 171A. The outer cylindrical part 171A and the inner cylindrical part 171B function as a slit valve holding part that holds the slit valve 106 that blocks the opening of the insertion port 131. In this embodiment, the slit valve 106, which is formed of an elastic material such as rubber or elastomer, has a middle section 161 that has a slit and blocks the opening of the insertion port 131, and a skirt-shaped section 162 that surrounds the middle section 161. The skirt-shaped section 162 protrudes downward from the lower surface of the middle section 161, reaching the upper end of the middle housing 172. A narrow portion is formed between the middle section 161 and the skirt-shaped section 162.

An upper lug that is a U-shaped folded portion protruding downward is formed at an upper end portion of the outer cylindrical part 171A, and a lower lug that protrudes upward is formed at an un upper end portion of the inner cylindrical part. The upper lug and the lower lug sandwich the narrow portion of the slit valve 106. The skirt-shaped section of the slit valve 106 is sandwiched between the outer cylindrical part 171A and the inner cylindrical part 171B. Until the male luer has penetrated through the slit valve, the slit valve is deformed downward with the slit valve sandwiched between the male luer and the plunger valve, so that a load is applied to the outer peripheral section of the slit valve. In the case in which the slit valve 106 is fixed by being simply sandwiched vertically, a load applied when the male luer is inserted may cause the slit valve to be detached inward from between the upper lug and the lower lug.

In order to reduce the detachment of the slit valve 106, a portion of the slit valve 106 outward of the sandwiched narrow portion is typically enlarged. However, in the case in which the diameter of the portion outward of the narrow portion is increased, the outer diameter of the insertion port 131 increases. To avoid this, in this embodiment, the slit valve 106 is provided with the skirt-shaped section 162 extending downward, and the skirt-shaped section 162 is sandwiched and fixed between the outer cylindrical part 171A and the inner cylindrical part 171B. As a result, an increase in the outer diameter of the insertion port 131 can be reduced, and the detachment of the slit valve 106 can be reduced. Furthermore, because the inside of the inner cylindrical part 171B is configured to serve as a space in which the plunger valve is moved, an increase in size in the height direction can be reduced.

The inner diameter of the inner cylindrical part 171B is substantially equal to the outer shape of the head section 111 of the plunger valve 101. Therefore, the inner cylindrical part 171B functions as a guide that when the head section 111 is pressed by the male connector 201, allows the head section 111 to move downward while reducing deformation of the head section 111. Therefore, the plunger valve 101 can be smoothly deformed. In addition, when the plunger valve 101 restores, the head section 111 is also guided, resulting in smooth restoration.

In this embodiment, an example in which a slit valve having a skirt-shaped section has been described. Alternatively, a typical disc-shaped slit valve may be used. Still alternatively, instead of providing a separate slit valve, the opening of the insertion port may be sealed by the plunger valve in a manner that allows the opening of the insertion port to open.

The radial flow path 155 that is formed in the outlet port housing 173 to connect the housing groove 153 and the axial flow path 156 together is shaped such that the diameter thereof is greater at the housing groove 153 than that at the axial flow path 156, and gradually becomes smaller toward the axial flow path 156. This structure allows acceleration of the fluid flowing in the radial flow path 156, and therefore, the direction of the flow can be smoothly changed to the axial direction in the axial flow path 156. Therefore, the stagnation is less likely to occur in the housing lower portion flow path 157. In addition, in this embodiment, two radial flow paths 156 are formed and separated from each other by 180° circumferentially. Therefore, the liquids flowing from the two radial flow paths 156 collide with each other with greater force in the axial flow path 156, and therefore, flow while being agitated in the axial flow path 156. It should be noted that the number of radial flow paths 156 is not limited to two.

In this embodiment, the outlet port housing 173 has a coupler 177 having a female threaded portion 184. The coupler 177 may be optionally formed, and may not be formed. In addition, although an example in which the outlet port 132 is a male connector has been described, the outlet port 132 is not limited to a male connector, and may be a female connector, or may be integrated with a tube, catheter, or the like.

Three types of samples were produced in which the notched grooves 151 were separated from the respective housing grooves 153 by 0°, 45°, and 90° circumferentially with the plunger valve 101 housed in the housing 103. The flow rate of each sample was assessed. It should be noted that the width W1 and the depth D of the upper end portion of the notched groove 151 were about 1.5 mm and about 2.5 mm, respectively. As a comparative example, a sample was produced which has four grooves having a width of about 0.5 mm and a depth of about 0.5 mm, and the flow rate of the sample was assessed.

The flow rate was measured as follows. A constant level tank was prepared in accordance with a flow rate test standard (JIS (Japanese Industrial Standards) T3223: Sterile, single-use intravascular catheters-over-needle peripheral catheters Annex F), which had a static hydraulic head height of 1000 ± 5 mm and a flow rate of 525 ± 25 mL/min before being connected to a sample. A male connector was connected to each sample, and the flow amount per minute was determined by weight measurement. Three samples were produced for each type, and measurement was conducted three times for each sample.

In the case in which the notched groove 151 was aligned with the housing groove 153 (0°), the flow rate was 127 mL/min. In the case in which the notched groove 151 was separated from the housing groove 153 by 45°, the flow rate was 124 mL/min; and by 90°, 125 mL/min. Meanwhile, for the comparative example in which the grooves had a uniform depth, the flow rate was 60 mL/min. The use of the plunger valve 101 having the notched grooves 151 can ensure a higher flow rate without application of high pressure. In addition, even when the notched grooves 151 were circumferentially separated from the housing grooves 153, any significant decrease in the flow rate was not observed.

For the three types of samples in which the notched grooves 151 were separated from the housing grooves 153 by 0°, 45°, and 90° circumferentially, the stagnation was assessed by the following method. The housing 103 of each sample was formed of a transparent material. The flow path of the needleless connector was filled with water colored with red ink. Thereafter, 2.5 mL of water that was not colored was injected into the needless connector using a syringe. The degree of coloration was visually observed. The injection using a syringe was completed in about 10 seconds.

In the case in which the notched grooves 151 were aligned with the housing grooves 153 (0°), a slight residue of the colored water was observed at the position where the middle section space 152 is formed. Meanwhile, in the cases in which the notched grooves 151 were separated from the housing grooves 153 by 45° and 90°, no residue of the colored water was observed in the needleless connector.

### INDUSTRIAL APPLICABILITY

The needleless connector according to the present disclosure can overcome various problems with conventional needleless connectors, and therefore, is useful in the medical field.

### DESCRIPTION OF REFERENCE CHARACTERS

- 101: PLUNGER VALVE
- 103: HOUSING
- 106: SLIT VALVE
- 111: HEAD SECTION
- 112: MIDDLE SECTION
- 113: CYLINDRICAL WALL SECTION
- 122: CAVITY
- 131: INSERTION PORT
- 132: OUTLET PORT
- 151: NOTCHED GROOVE
- 152: MIDDLE SECTION SPACE
- 153: HOUSING GROOVE
- 155: RADIAL FLOW PATH
- 156: AXIAL FLOW PATH
- 157: HOUSING LOWER PORTION FLOW PATH
- 161: MIDDLE SECTION
- 162: SKIRT-SHAPED SECTION
- 171: INSERTION PORT HOUSING
- 171A: OUTER CYLINDRICAL PART
- 171B: INNER CYLINDRICAL PART
- 172: MIDDLE HOUSING
- 173: OUTLET PORT HOUSING
- 175: BASE SECTION
- 176: SUPPORTING WALL
- 177: COUPLER
- 178: VENTILATION HOLE
- 181: MALE THREADED PORTION
- 182: PROTRUSION SECTION
- 183: POSITIONING PROJECTION
- 184: FEMALE THREADED PORTION
- 201: MALE CONNECTOR
- 211: RANGE

## Claims

1. A needleless connector comprising:
a cylindrical housing having, at an end portion thereof, an insertion port into which a male connector is inserted; and
a plunger valve housed in the housing,
wherein
a flow path is formed between an inner wall surface of the housing and an outer wall surface of the plunger valve,
the plunger valve has a cylindrical wall section, a head section formed closer to the insertion port than is the cylindrical wall section, and a middle section formed between the head section and the cylindrical wall section,
the plunger valve is configured to be deformed such that the volume of the flow path increases, when a male connector is inserted in the insertion port, and restore such that the volume of the flow path decreases, when a male connector inserted in the insertion port is removed,
the flow path has an upstream flow path formed at the position of the head section, a downstream flow path formed at the position of the cylindrical wall section, and a middle flow path connecting the upstream flow path and the downstream flow path together, and
the head section has a notched groove having a radial depth that gradually becomes smaller from a top vertex portion thereof toward the middle section.

2. The needleless connector of claim 1, wherein
the notched groove has a circumferential width that gradually becomes narrower from the top vertex portion toward the middle section.

3. A needleless connector comprising:
a cylindrical housing having, at an end portion thereof, an insertion port into which a male connector is inserted; and
a plunger valve housed in the housing,
wherein
a flow path is formed between an inner wall surface of the housing and an outer wall surface of the plunger valve,
the plunger valve has a cylindrical wall section, a head section formed closer to the insertion port than is the cylindrical wall section, and a middle section formed between the head section and the cylindrical wall section,
the plunger valve is configured to be deformed such that the volume of the flow path increases, when a male connector is inserted in the insertion port, and restore such that the volume of the flow path decreases, when a male connector inserted in the insertion port is removed, and
the flow path has a portion in which a liquid flows circumferentially.

4. The needleless connector of claim 3, wherein
the flow path has an upstream flow path formed at the position of the head section, a downstream flow path formed at the position of the cylindrical wall section, and a middle flow path connecting the upstream flow path and the downstream flow path together, and
the upstream flow path and the downstream flow path are separated from each other circumferentially.

5. The needleless connector of claim 4, wherein
the head section has a notched groove serving as the upstream flow path,
the housing has a housing groove serving as the downstream flow path at a portion thereof opposite the cylindrical wall section, and
the plunger valve is housed in the housing with the notched groove and the housing groove separated from each other circumferentially.

6. A needleless connector comprising:
a cylindrical housing having, at an end portion thereof, an insertion port into which a male connector is inserted;
a plunger valve housed in the housing; and
a slit valve arranged closer to the insertion port than is the plunger valve and configured to block an opening of the insertion port,
wherein
a flow path is formed between an inner wall surface of the housing and an outer wall surface of the plunger valve,
the plunger valve has a cylindrical wall section, a head section formed closer to the insertion port than is the cylindrical wall section, and a middle section formed between the head section and the cylindrical wall section,
the plunger valve is configured to be deformed such that the volume of the flow path increases, when a male connector is inserted in the insertion port, and restore such that the volume of the flow path decreases, when a male connector inserted in the insertion port is removed,
the slit valve has a middle section having a slit and configured to block the opening of the insertion port in a manner that allows the insertion port to be openable and closeable, and a skirt-shaped section formed on an outer peripheral side of the middle section and protruding from the middle section toward the plunger valve,
the housing has, at the insertion port, an outer cylindrical part and an inner cylindrical part, and
the inner cylindrical part, together with the outer cylindrical part, sandwiches the skirt-shaped section, and is positioned between the skirt-shaped section and the head section and configured to guide movement of the head section.
